# FASCICULE DE BREVET EUROPEEN

(11) **EP 2 575 828 B1**
(45) Date de publication et mention de la délivrance du brevet: **23.08.2017**
(21) Numéro de dépôt: 11725137.1
(22) Date de dépôt: 09.05.2011
(51) Int. Cl.: A61K 9/00, A61K 33/00, A61P 25/30, A61P 25/32

(54) **MÉDICAMENT INHALABLE À BASE DE XÉNON POUR PRÉVENIR LES RECHUTES ADDICTIVES CHEZ L'ÊTRE HUMAIN**
XENONBASIERTES INHALIERBARES ARZNEIMITTEL ZUR VORBEUGUNG VON SUCHTRÜCKFÄLLEN BEI MENSCHEN
XENON-BASED INHALABLE DRUG FOR PREVENTING ADDICTION RELAPSES IN HUMANS

(30) Priorité: 03.06.2010 FR 1054333
(43) Date de publication de la demande: 10.04.2013
(73) Titulaire: L'Air Liquide Société Anonyme pour l'Etude et l'Exploitation des Procédés Georges Claude, 75007 Paris (FR)
(72) Inventeur: BESSIERE, Baptiste, F-92130 Issy Les Moulineaux (FR); PYPE, Jan, B-1540 Herne (BE)
(74) Mandataire: Pittis, Olivier
(86) Numéro de dépôt international: PCT/FR2011/051038
(87) Numéro de publication internationale: WO 2011/151551

(56) Documents cités:
- US-A1- 2005 124 963
- DAVID ET AL: "Nitrous Oxide and Xenon Prevent Amphetamine-Induced Carrier-Mediated Dopamine Release in a Memantine-Like Fashion and Protect Against Behavioral Sensitization", BIOLOGICAL PSYCHIATRY, ELSEVIER SCIENCE, NEW YORK, NY, US LNKD- DOI:10.1016/J.BIOPSYCH.2005.10.007, vol. 60, no. 1, 1 juillet 2006 (2006-07-01), pages 49-57, XP005512613, ISSN: 0006-3223
- ABRAINI J H ET AL: "Potentially neuroprotective and therapeutic properties of nitrous oxide and xenon", ANNALS OF THE NEW YORK ACADEMY OF SCIENCES 2005 US LNKD- DOI:10.1196/ANNALS.1344.025, vol. 1053, 2005, pages 289-300, XP002602875, ISSN: 0077-8923
- WOLF MARINA E: "The role of excitatory amino acids in behavioral sensitization to psychomotor stimulants", PROGRESS IN NEUROBIOLOGY (OXFORD), vol. 54, no. 6, avril 1998 (1998-04), pages 679-720, XP002602876, ISSN: 0301-0082
- NAKATA YOSHINORI ET AL: "Minimum alveolar concentration (MAC) of xenon with sevoflurane in humans", ANESTHESIOLOGY (HAGERSTOWN), vol. 94, no. 4, April 2001 (2001-04), pages 611-614, ISSN: 0003-3022

## Description

La présente invention porte sur une composition gazeuse, c'est-à-dire un médicament gazeux, à base de xénon gazeux administrable par inhalation pour prévenir, éviter ou réduire la vulnérabilité à la rechute d'un patient traité pour une dépendance ou une addiction, notamment à une ou plusieurs drogues et/ou à l'alcool, c'est-à-dire y compris les patients humains ayant été sujets à des poly-addictions, c'est-à-dire la prise de plusieurs produits psychotropes en même temps.

La dépendance, encore appelée addiction, à un (ou plusieurs) produit psychotrope ou analogue, tel que drogue ou alcool, est un trouble chronique se caractérisant par un besoin compulsif de ce produit psychotrope ou analogue, une perte du contrôle de la quantité de produit prise et une émergence d'états émotionnels négatifs lorsque le produit psychotrope n'est pas disponible ou que l'accès audit produit est empêché.

Une addiction est donc, en d'autres termes, une envie répétée et irrépressible de la prise d'un produit psychotrope en dépit des efforts et de la motivation du sujet à s'y soustraire.

Il a été reporté par G.F. Koob et coll., dans l'article intitulé : Development of pharmacotherapies for drug addiction: a Rosetta stone approach. ; Nat. Rev. Drug Discov. 2009 ; 8:500-515, que des rechutes sont observées chez plus de 90% des patients ayant été traités pour dépendance à un produit psychotrope du type alcool, nicotine, cocaine, héroïne, opioïdes ou analogues, dans l'année qui suit la fin du traitement ayant conduit au sevrage desdits patients.

Réussir à empêcher les rechutes est fondamental si l'on souhaite obtenir une rémission totale du patient, c'est-à-dire une désaccoutumance totale sur une longue période.

Le document US - A - 2005/0124963 propose de traiter divers aspects de l'alcoolisme, en particulier les rechutes alcooliques, par administration d'un agent anesthésique, tel l'isoflurane, l'halothane ou le N₂O, jusqu'à obtenir une anesthésie générale profonde du patient.

Or, actuellement, il n'existe pas de traitement efficace ciblant les symptômes au coeur de l'addiction, c'est-à-dire l'envie et la sensation de manque de produit psychotrope après une période d'abstinence prolongée.

Le problème à résoudre est dès lors de proposer une composition médicamenteuse ou pharmaceutique permettant de diminuer la sensation de manque chez des patients sevrés de manière à éviter que ceux-ci ne rechutent et ne recommencent à s'administrer ou à ingérer un ou plusieurs produits psychotropes.

La solution proposée concerne alors une composition gazeuse contenant du xénon gazeux pour une utilisation pour prévenir une rechute d'un patient ayant été sevré d'un produit psychotrope ayant conduit à une accoutumance chez ledit patient, le xénon étant administré au patient par inhalation et contenant une proportion volumique efficace de xénon comprise entre au moins 10 % et moins de 50% en volume.

Le patient est un être humain, c'est-à-dire un homme ou une femme, y compris les enfants, les adolescents, ou tout autre groupe d'individus.

Selon le cas, la composition gazeuse (i.e. le médicament gazeux) inhalable selon l'invention peut comprendre l'une ou plusieurs des caractéristiques suivantes :
- elle contient moins de 40% en volume de xénon, de préférence moins de 30% en volume de xénon.
- elle contient en outre de l'oxygène, de préférence au moins 21% en volume d'oxygène.
- elle contient en outre un composé additionnel choisi dans le groupe formé par N₂O, Ar, Kr, He, Ne, NO, CO, H₂S et N₂.
- le produit psychotrope est une drogue ou de l'alcool, en particulier l'héroïne, la nicotine, le cannabis, la cocaïne ou les amphétamines, y compris des dérivés de ces produits.
- le xénon est administré au patient par inhalation une ou plusieurs fois par jour, pendant une durée totale de traitement de plusieurs jours à plusieurs années.
- le xénon gazeux est mélangé avec un gaz contenant de l'oxygène, en particulier le xénon est mélangé avec de l'air ou un mélange N₂/O₂.
- le patient a été sevré de plusieurs produits psychotropes ayant conduit à des accoutumances chez ledit patient.

Le xénon gazeux selon l'invention sert donc à fabriquer une composition médicamenteuse inhalable destinée à prévenir une rechute d'un patient ayant été sevré d'un produit psychotrope ou de plusieurs produits psychotropes (i.e. cas des polytoxicomanies) ayant conduit à une accoutumance chez ledit patient, ladite composition médicamenteuse pouvant comprendre tout ou partie des caractéristiques susmentionnées.

Dit autrement, selon l'invention, on administre par inhalation du xénon gazeux à un patient, c'est-à-dire un homme, une femme ou un enfant, de manière à prévenir une rechute dudit patient ayant été sevré d'un produit psychotrope ou de plusieurs produits psychotropes ayant conduit à une accoutumance chez ledit patient.

En fait, une hypothèse prépondérante dans le domaine de l'addiction aux drogues ou aux autres produits analogues ou l'alcoolisme, est que le système glutamatergique est impliqué de manière critique dans les comportements addictifs des patients, en particulier lors des rechutes comme expliqué par J.T. Gass et coll., dans Glutamatergic substrates of drug addiction and alcoholism ; Biochem Pharmacol ; 2008 ;75:218-265.

En effet, la stimulation des récepteurs mGluR2/3 permet bloquer la reprise de la cocaïne, de la nicotine et de l'alcool, probablement en réduisant la libération du glutamate des terminaisons presynaptiques, apportant davantage de preuves que c'est bien la libération de glutamate qui gouverne la rechute.

De façon similaire, les antagonistes des récepteurs mGluR du groupe I bloquent la reprise de la recherche de cocaïne, de nicotine et d'alcool, comme expliqué par L.A. Knackstedt et coll. ; Glutamate and reinstatement. Curr Opin Pharmacol 2009;9:59-64.

Dans des études utilisant l'acamprosate, un médicament,connu pour réduire un système hyper-glutamatergique, on a pu constater une réduction des effets délétère (augmentation de la prise d'alcool) suite à la privation d'alcool chez des rats alcool-dépendants, comme rappelé par Spanagel et al. ; Acamprosate and alcohol: I. Effects on alcohol intake following alcohol deprivation in the rat. Eur J Pharmacol 1996,305:39-44.

Etant donné que des structures différentes du cerveau contribuent à la reprise des différentes classes de drogues et d'alcool, les neuroadaptations glutamatergiques induites par la drogue et les effets pharmacologiques du glutamate sur cette reprise, qui a été d'abord identifiée dans le noyau accumbens, peuvent être généralisées à d'autres structures clés du cerveau, telles que les aires tegmentales ventrales ou l'amygdale.

Néanmoins, le rôle de la transmission glutamatergique au niveau du noyau accumbens est indéniable et important pour ce qui est de la reprise du besoin de recherche de drogue et ce, quelle que soit la classe de drogue considérée. En d'autre terme, le maintien de l'homéostasie glutamatergique semble donc une cible pharmaco-thérapeutique de choix dans le traitement de la rechute chez le patient accoutumé.

Puisque le xénon a montré des propriétés inhibitrices des voies de signalement glutamatergique excitatoire, d'abord via son action sur les récepteurs *N*-méthyl D-aspartate (NMDA), mais aussi sur les récepteurs α-amino-3-hydroxy-5-méthylisoazol-4-propionate (AMPA) et les récepteurs kainate, on propose maintenant avec la présente invention d'utiliser le xénon inhalé pour prévenir ou pour réduire la vulnérabilité des patients à la rechute à la prise de drogues et d'alcool, c'est-à-dire de produits psychotropes engendrant une dépendance.

Le document US2005/0124963 enseigne que le N₂O à forte concentration ou tout autre agent anesthésique gazeux similaire pourrait être utilisé pour dans le traitement de l'alcoolisme et de l'addiction. Cependaant, le xénon n'est pas mentionné dans ce document. De plus, bien que des similarités existent entre le N₂O et le xénon, de nombreuses disparités existent aussi entre ces deux gaz, tant pour ce qui concerne leur pouvoir anesthésique que leur pouvoir analgésique et leur effet neuroprotecteur... Ainsi, l'étude de Valeggi et all., intitulé « Xénon up regulates several genes that are not up-regulated by nitrous oxide », J. Neurosurg. Anesthesiol. 2008, 20 : 226-32, démontre clairement les différences de mécanisme d'action entre ces deux gaz. Ces données rendent comptent des différences d'effet pharmacologique entre le N₂O et le xénon. Dès lors, une observation faite pour le N₂O n'est nullement extrapolable, de manière évidente, au xénon. D'ailleurs, comme montré dans les exemples comparatifs ci-après, les résultats d'efficacité obtenus avec du xénon sont bien plus notables que ceux obtenus avec du N₂O.

D'une façon générale, lors du traitement dans le cadre de la présente invention, l'administration de xénon peut se faire via un moyen d'administration classique, tel un ventilateur, un nébuliseur ou spontanément avec des bouteilles de gaz préconditionnées, ledit moyen d'administration étant raccordé à un masque facial ou nasal, ou des lunettes nasales.

La durée d'administration est choisie au cas par cas en fonction de l'importance de l'état de manque affectant le patient considéré, par exemple le xénon pourra être administré pendant une durée d'administration de quelques minutes à quelques dizaines de minutes, voire d'heures, par exemple moins d'une heure, à une fréquence pouvant atteindre une à plusieurs fois par jour ou par semaine et sur une durée totale de traitement de un ou plusieurs jours, semaines, mois ou années, par exemple 1 fois par jour pendant 6 mois.

Le xénon ou mélange gazeux à base de xénon est préférentiellement conditionné en bouteille de gaz sous pression ou sous forme liquide, par exemple dans une bouteille d'un à plusieurs litres (contenance en eau), notamment entre 1 et 50 litres, et/ou à une pression comprise entre 2 et 300 bar.

Le xénon ou mélange gazeux à base de xénon peut se présenter sous forme « prêt à l'emploi », par exemple en pré-mélange avec de l'oxygène, ou alors être mélangé sur site lors de son utilisation, notamment avec de l'oxygène et éventuellement un autre composé gazeux, par exemple de l'azote.

L'invention va maintenant être mieux comprise grâce aux Exemples comparatifs suivants donnés en références aux Figures annexées parmi lesquelles :
- la Figure 1 schématise le protocole sur l'effet de privation d'alcool (EPA) chez le rat ;
- la Figure 2 montre les effets du xénon sur la consommation d'alcool après privation (modèle EPA) ;
- la Figure 3 montre les effets du protoxyde d'azote (N₂O) sur la consommation d'alcool après privation (modèle EPA) ; et
- la Figure 4 schématise le protocole de rechute alcoolique stimuli-induite chez le rat ;
- la Figure 5 montre les effets du xénon et du protoxyde d'azote (N₂O) sur la rechute alcoolique stimuli-induite.

### Exemples comparatifs

Les essais pré-cliniques exposés ci-après ont été réalisés pour démontrer les effets positifs d'une inhalation de xénon par des rats ayant subi un sevrage alcoolique et qui sont susceptibles de subir une rechute. A titre comparatif l'effet du protoxyde d'azote (mélange 50%-50% vol. N₂O/O₂) sera aussi évalué.

Deux modèles validés pour tester des traitements dans la rechute alcoolique chez le rat ont été utilisés (Sanchis-Segura and Spanagel, Behavioural assessment of drug reinforcement and addictive features in rodents : an overview, Addiction Biology 11, 2-38, 2006):
(1) Modèle de privation d'acool (EPA)
(2) Modèle de rechute alcoolique stimuli-induite

### 1. Modèle de privation d'acool (EPA)

Le modèle de privation d'acool (EPA) consiste à habituer les animaux à une consommation d'alcool pendant une période de 8 semaines, suivie d'une période de privation de 2 semaines, pendant laquelle l'alcool a été retiré. Ce cycle est reproduit 8 fois.

Après une période de privation, l'accès à de l'alcool provoque une augmentation importante mais temporaire de la consommation d'alcool par rapport au niveau de référence. Ce modèle est idéal pour étudier le comportement de la rechute. Il a été validé avec l'acamprosate et la naltrexone. Cette validation pharmacologique montre la valeur prédictive de ce modèle et nous permet de caractériser plus précisément les présumés médicaments anti-rechute ainsi que les mécanismes neurobiologiques des comportements addictifs.

Le protocole expérimental suivant a été suivi.

16 rats mâles Wistar ont un accès libre et continu à 2 biberons, l'un contenant de l'eau du robinet et l'autre contenant de l'éthanol à 5%, 10% et 20% (vol./ vol.) dans leurs cages.

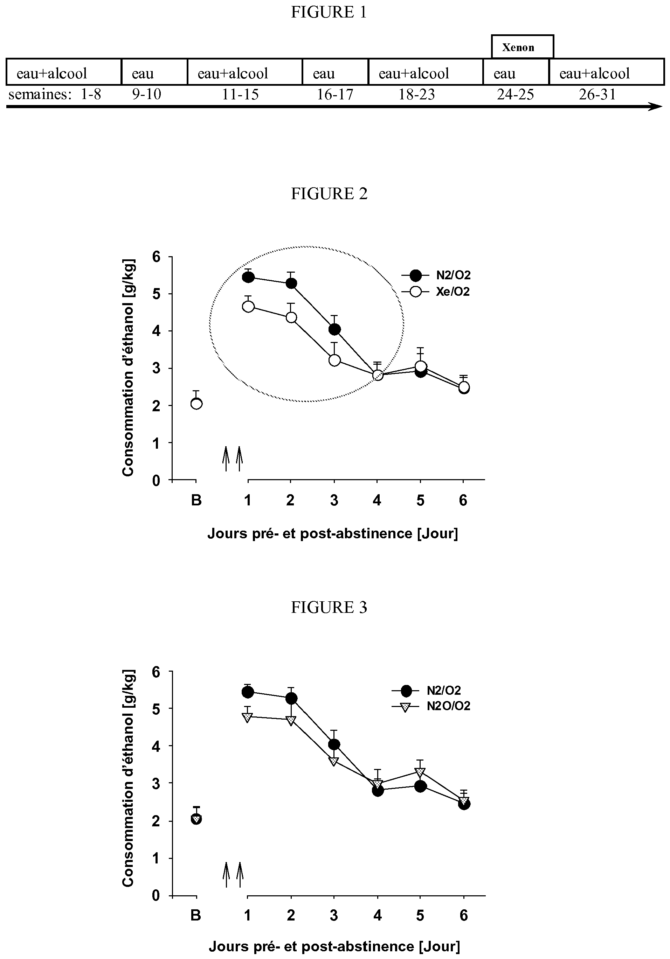

La première période de privation de 2 semaines est mise en place après 8 semaines de disponibilité de l'alcool en continu, comme illustré en Figure 1.

Après cette période de privation, les rats auront accès à l'alcool et à nouveau à au moins 2 périodes de privation introduites de manière aléatoire.

Tous les animaux sont répartis en 2 groupes (8 rats/groupe) de telle manière que pour la ligne de base moyenne (semaine 23), la consommation d'alcool totale sera sensiblement la même dans chaque groupe.

Au cours de la dernière journée de la semaine 25 à environ 9h, tous les animaux sont exposés au mélange gazeux Xe/O₂ (50% / 50%) pendant 1 heure.

Le groupe témoin est exposé à N₂/O₂ (50% / 50%). Le lendemain, à 9 h tous les animaux sont exposés à ces mêmes mélanges gazeux une seconde fois pendant 1 heure.

Les expositions au mélange gazeux sont représentées par deux flèches dans la Figure 2.

Suite à cette dernière exposition les bouteilles d'alcool seront offertes à nouveau (jours post-abstinence).

À la suite des exposions aux différents gaz, la consommation d'éthanol totale (g/kg de poids corporel) est mesurée quotidiennement à environ 10 heures, pendant 6 jours.

Comme on le voit sur la Figure 2, suite à la période d'abstinence, les rats ont fortement augmenté leur consommation d'alcool comme classiquement reporté dans la littérature avec ce modèle. Ainsi, ils passent d'une consommation d'alcool d'environ 2 g/kg par jour (point B sur la Figure 2) à une consommation d'environ 5,5 g/kg un jour après la période d'abstinence.

De façon intéressante, l'effet du xénon sur la privation alcoolique se traduit par une réduction de la consommation alcoolique suite au traitement par le mélange contenant du xénon. En effet, la courbe de consommation du groupe Xe représentée par les ronds blancs et en dessous du groupe contrôle en rond noir.

Le calcule des aires sous la courbe (qui permet de comparer un ensemble de points) met en évidence une valeur de 14.28 ± 1.3 g/kg*jour pour le groupe Xe inférieur au groupe contrôle N₂/O2 qui a une valeur de 16.34±0.9 g/kg*jour. Une différence notable de deux unités en faveur d'un effet du xénon est donc observée.

A titre comparatif, la Figure 3 montrent que, dans des mêmes conditions, le N₂O réduit dans une bien moindre mesure (triangle gris) cette consommation d'alcool.

En effet, le groupe N₂O conduit à une aire sous la courbe de 15.34±1.3 g/kg*jour comparativement au groupe contrôle qui a une aire sous la courbe de 16.34±0.9 g/kg*jour. La différence est ici seulement de 1 point suggérant un effet plus faible avec le N₂O.

En résumé, le traitement par inhalation de xénon va permettre de réduire ce rebond de consommation d'alcool démontrant ainsi les propriétés anti-rechute addictives du xénon inhalé.

### 2. Modèle de rechute alcoolique stimuli-induite

Un autre modèle animal bien établi dans la littérature a été utilisé pour évaluer les effets anti-rechute du xénon, à savoir le modèle de rechute alcoolique stimuli-induite.

Ce modèle est basé sur le fait que la prise de drogues ou d'alcool susceptible d'entraîner une dépendance est associée à de nombreux stimuli environnementaux (auditif, olfactif, visuel) susceptibles d'entraîner, eux-mêmes, une rechute.

Ce modèle est particulièrement intéressant car il se rapproche des conditions réelles chez l'homme puisque les personnes ayant été sevrées rechutent souvent du fait de stimuli environnementaux. Les résultats obtenus avec ce modèle sont donc particulièrement importants.

Brièvement, les expériences ont été réalisées dans des chambres de conditionnement opérant équipées de 2 leviers permettant la distribution de l'eau ou de l'alcool.

24 rats mâles Wistar ont été utilisés. Les animaux sont formés à l'auto-administration d'éthanol à 10% en volume au cours de sessions quotidiennes d'une durée de 30 minutes.

Au cours de la phase de conditionnement, les animaux sont formés à distinguer la disponibilité de l'alcool et de l'eau. Le stimulus-1 (extrait d'orange) est le signal de la disponibilité de l'alcool et chaque appui sur le levier n°1 se traduit par le stimulus conditionné n°1 (lumière clignotante) et la distribution immédiate d'alcool (30 µl). Par ailleurs, le levier n°2 reste inactif pendant les « sessions alcool» tout au long de l'expérience.

Le stimulus n° 2 (extrait d'anis) donnera le signal de disponibilité de l'eau lors des « sessions eau » et chaque appuie sur le levier n°2 se traduit par la présentation du stimulus conditionné n°2 (lumière constante) et la distribution d'eau (30 µl). Le conditionnement à l'eau est utilisé pour mesurer la sélectivité du traitement médicamenteux. Le levier n°1 reste inactif pendant les "sessions eau" tout au long de l'expérience.

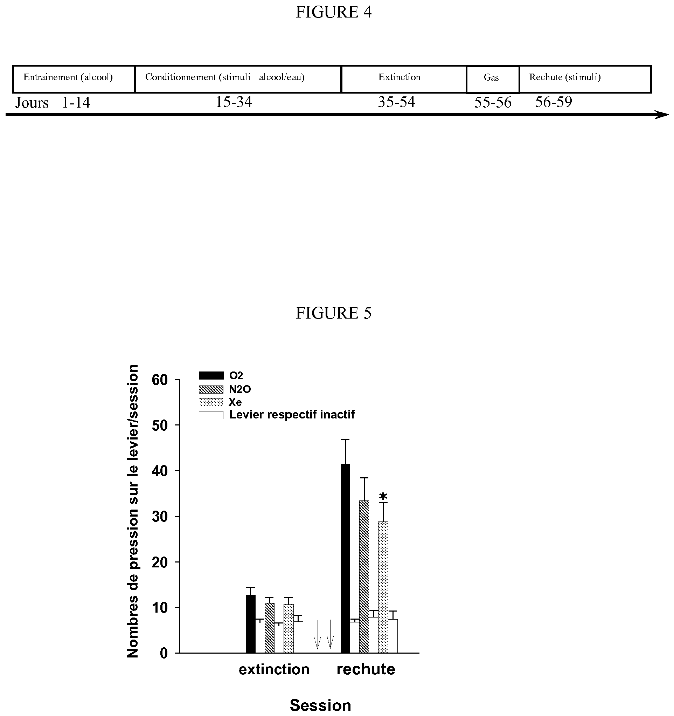

Les sessions d'alcool et d'eau (une session de 30 min/jour) sont effectuées de façon aléatoire afin d'obtenir un total de 10 sessions d'alcool et de 10 session d'eau (voir le schéma Figure 4).

Ensuite, la phase d'extinction débute par la présentation des leviers sans association avec les stimuli olfactifs. Les réponses à l'appuie des leviers ne donnent pas lieu à la mise à disposition ni de l'alcool, ni de l'eau.

Enfin, au cours de la phase de rechute, les rats sont exposés aux mêmes conditions que lors de la phase de conditionnement, à ceci près que les liquides (eau ou d'alcool) ne leur sont pas distribués.

Afin de tester l'effet des gaz, et en particulier du xénon, sur la rechute alcoolique stimuli-induite, les animaux sont répartis en trois groupes (8 rats/groupe) sur la base de leurs performances au cours des quatre dernières séances de conditionnement. Une exposition au gaz de 1 heure est effectuée 24 heures (jour 55) et 3 heures avant le test de rechute (jour 56). Les expositions aux gaz sont indiquées par deux flèches sur la Figure 5.

Comme le montre la Figure 5, les animaux traités par le xénon présentent une diminution significative du nombre pressions sur le levier délivrant l'alcool suite à l'exposition au stimulus-1 comparativement au groupe contrôle respirant le mélange N₂/O₂ 50%/50% (p < 0,05). A l'inverse, aucun effet significatif n'a été observé avec le N₂O.

Ces données démontrent un effet anti-rechute très net lié au traitement au xénon inhalé mais, à l'inverse, démontre que l'effet n'est pas avéré avec le N₂O, en particulier lorsque des stimuli extérieurs sont associés à la prise d'alcool.

Au final, l'ensemble de ces résultats démontrent les propriétés préventives anti-rechute du xénon dans deux modèles classiquement utilisés dans cette pathologie.

## Revendications

1. Composition gazeuse contenant du xénon gazeux pour une utilisation pour prévenir une rechute d'un patient ayant été sevré d'au moins un produit psychotrope ayant conduit à une accoutumance chez ledit patient, ledit patient étant un être humain, le xénon étant administré au patient par inhalation et contenant une proportion volumique efficace de xénon comprise entre au moins 10% et moins de 50% en volume.

2. Composition pour une utilisation selon la revendication précédente, **caractérisée en ce qu'**elle contient moins de 40% en volume de xénon.

3. Composition pour une utilisation selon l'une des revendications précédentes, **caractérisée en ce qu'**elle contient moins de 30% en volume de xénon.

4. Composition pour une utilisation selon l'une des revendications précédentes, **caractérisée en ce qu'**elle contient en outre de l'oxygène

5. Composition pour une utilisation selon l'une des revendications précédentes, **caractérisée en ce qu'**elle contient de l'ordre de 21% en volume d'oxygène.

6. Composition pour une utilisation selon l'une des revendications précédentes, **caractérisée en ce qu'**elle contient en outre un composé additionnel choisi dans le groupe formé par N₂O, Ar, Kr, Ne, He, Ne, NO, CO, H₂S et N₂.

7. Composition pour une utilisation selon l'une des revendications précédentes, **caractérisée en ce que** le produit psychotrope est une drogue ou de l'alcool, en particulier l'héroïne, la cocaïne, la nicotine, le cannabis ou les amphétamines.

8. Composition pour une utilisation selon l'une des revendications précédentes, **caractérisée en ce que** le xénon est administré au patient par inhalation une ou plusieurs fois par jour, pendant une durée totale de traitement de plusieurs jours à plusieurs années.

9. Composition pour une utilisation selon l'une des revendications précédentes, **caractérisée en ce que** le xénon est administré au patient une ou plusieurs fois par jour pendant une durée d'inhalation de quelques minutes à une ou plusieurs heures.

10. Composition pour une utilisation selon l'une des revendications précédentes, **caractérisée en ce que** le patient a été sevré de plusieurs produits psychotropes ayant conduit à des accoutumances chez ledit patient.

11. Composition pour une utilisation selon l'une des revendications précédentes, **caractérisée en ce que** le xénon ou mélange gazeux à base de xénon est conditionné en bouteille de gaz à une pression comprise entre 2 et 300 bar.

## Patentansprüche

1. Gaszusammensetzung, enthaltend Xenongas, für eine Verwendung zum Verhindern eines Rückfalls eines Patienten, der von mindestens einem psychotropen Produkt, das bei dem Patienten zu einer Abhängigkeit führte, entwöhnt worden ist, wobei der Patient ein menschliches Wesen ist, wobei das Xenon dem Patienten durch Inhalation verabreicht wird und einen wirksamen Volumenanteil Xenon, umfasst zwischen mindestens 10 Volumen-% und mindestens 50 Volumen-%, enthält.

2. Zusammensetzung für eine Verwendung nach dem vorstehenden Anspruch, **dadurch gekennzeichnet, dass** sie mindestens 40 Volumen-% Xenon enthält.

3. Zusammensetzung für eine Verwendung nach einem der vorstehenden Ansprüche, **dadurch gekennzeichnet, dass** sie mindestens 30 Volumen-% Xenon enthält.

4. Zusammensetzung für eine Verwendung nach einem der vorstehenden Ansprüche, **dadurch gekennzeichnet, dass** sie des Weiteren Sauerstoff enthält

5. Zusammensetzung für eine Verwendung nach einem der vorstehenden Ansprüche, **dadurch gekennzeichnet, dass** sie Sauerstoff in der Größenordnung von 21 Volumen-% enthält.

6. Zusammensetzung für eine Verwendung nach einem der vorstehenden Ansprüche, **dadurch gekennzeichnet, dass** sie des Weiteren eine zusätzliche Verbindung, ausgewählt aus der Gruppe, gebildet aus N₂O Ar, Kr, Ne, He, Ne, NO, CO, H₂S und N₂ enthält.

7. Zusammensetzung für eine Verwendung nach einem der vorstehenden Ansprüche, **dadurch gekennzeichnet, dass** das psychotrope Produkt eine Droge oder Alkohol, insbesondere Heroin, Kokain, Nikotin, Cannabis oder Amphetamine ist.

8. Zusammensetzung für eine Verwendung nach einem der vorstehenden Ansprüche, **dadurch gekennzeichnet, dass** das Xenon dem Patienten einmal oder mehrere Male täglich durch Inhalation, während einer Gesamtdauer der Behandlung von mehreren Tagen bis mehreren Jahren, verabreicht wird.

9. Zusammensetzung für eine Verwendung nach einem der vorstehenden Ansprüche, **dadurch gekennzeichnet, dass** das Xenon dem Patienten einmal oder mehrere Male täglich während einer Inhalationsdauer von einigen Minuten bis zu einer oder mehreren Stunden, verabreicht wird.

10. Zusammensetzung für eine Verwendung nach einem der vorstehenden Ansprüche, **dadurch gekennzeichnet, dass** der Patient von mehreren psychotropen Produkten, die zu Abhängigkeiten bei dem Patienten führten, entwöhnt worden ist.

11. Zusammensetzung für eine Verwendung nach einem der vorstehenden Ansprüche, **dadurch gekennzeichnet, dass** das Xenon oder xenonbasierte Gasgemisch in Gasflaschen bei einem Druck, umfasst zwischen 2 und 300 bar, abgefüllt wird.

## Claims

1. Gas composition containing xenon gas for use in preventing the relapse of a patient who has been weaned from at least one psychotropic product which resulted in said patient becoming habituated, said patient being a human being, the xenon being administered to the patient by inhalation and containing an effective volume proportion of xenon between at least 10% and less than 50% by volume.

2. Composition for a use as claimed in the preceding claim, **characterised in that** it contains less than 40% by volume of xenon.

3. Composition for a use according to one of the preceding claims, **characterised in that** it contains less than 30% by volume of xenon.

4. Composition for a use according to one of the preceding claims, **characterised in that** it furthermore contains oxygen.

5. Composition for a use according to one of the preceding claims, **characterised in that** it contains about 21% by volume of oxygen.

6. Composition for a use according to one of the preceding claims, **characterised in that** it further contains an additional compound chosen from the group comprising N₂O, Ar, Kr, Ne, He, Ne, NO, CO, H₂S and N2.

7. Composition for a use according to one of the preceding claims, **characterised in that** the psychotropic product is a drug or alcohol, in particular heroin, cocaine, nicotine, cannabis or amphetamines.

8. Composition for a use according to one of the preceding claims, **characterised in that** xenon is administered to the patient by inhalation once or several time per day, during a total treatment period of several days to several years.

9. Composition for a use according to one of the preceding claims, **characterised in that** xenon is administered to the patient once or several times per day for an inhalation duration of a few minutes to one or several hours.

10. Composition for a use according to one of the preceding claims, **characterised in that** the patient has been weaned from several psychotropic products which resulted in said patient becoming habituated.

11. Composition for a use according to one of the preceding claims, **characterised in that** xenon or a xenon-based gaseous mixture is conditioned in a gas bottle at a pressure between 2 and 300 bar.
